# EUROPÄISCHE PATENTANMELDUNG

(11) **EP 2 333 515 A1**
(43) Veröffentlichungstag der Anmeldung: **15.06.2011**
(21) Anmeldenummer: 09015341.2
(22) Anmeldetag: 11.12.2009
(51) Int. Cl.: G01N 15/02, G01N 21/03, G01N 21/47, G01N 21/64, G01N 33/18

(54) **Mittel zur Detektion von lumineszierenden und/oder lichtstreuenden Partikeln in strömenden Flüssigkeiten**

(71) Anmelder: Bayer Technology Services GmbH, 51368 Leverkusen (DE)
(72) Erfinder: Hermansen, Christoph, 14715 Kotzen (DE)

(57) **Zusammenfassung**

Die Erfindung betrifft eine Sonde zur Detektion von lumineszierenden und / oder lichtstreuenden Partikeln in strömenden Flüssigkeiten, die eine Messzelle beinhaltend ein Rohrleitungskanal in dem die zu bemessene Flüssigkeit durchströmt, mindestens ein transparentes Fenster in einer Wand der Rohrleitung, mindestens eine Lichtquelle zur Erzeugung eines dimensionierten Anregungslichtstrahls, der durch das Fenster die lumineszierenden und / oder lichtstreuenden Partikeln in dem Rohrleitungskanal in einem optisch begrenzten Lichtvolumen anregt, mindestens einen Detektor, der durch das Fenster oder durch ein weiteres Fenster ein von den lumineszierenden und / oder lichtstreuenden Partikeln emittiertes Licht aufnimmt, aufweist, wobei die Messzelle so aufgebaut ist, dass der dimensionierte Anregungslichtstrahl und das emittierte Licht senkrecht zueinander orientiert sind und jedes Partikel sich innerhalb des Messvolumens parallel zum Flüssigkeitsstrom, geradlinig mit einem festem Winkel zum Anregungslicht bewegt. Die Erfindung betrifft außerdem ein Verfahren zur Detektion von lumineszierenden und / oder lichtstreuenden Partikeln in strömenden Flüssigkeiten sowie die Verwendung der erfindungsgemäßen Sonde und des Verfahren zur Online-Überwachung einer Produktionsanlage, insbesondere einer Kunststoffsproduktionsanlage oder einer Kläranlage.

## Beschreibung

Die Erfindung bezieht sich auf eine Sonde und ein Verfahren zur Detektion von lumineszierenden und / oder lichtstreuenden Partikeln in Flüssigkeiten, die in einer Rohrleitung strömen.

Bei der Produktion von Kunstoffen ist die Überwachung von Produktionsprozessen von entscheidender Bedeutung, um frühzeitige Informationen über die Produktqualität zu erlangen. Insbesondere ist die Anzahl von lumineszierenden bzw. fluoreszierenden Partikeln ein entscheidender Qualitätsfaktor für die Anwendbarkeit des Kunststoffs für die Herstellung von Fertigprodukten für optische Anwendungen insbesondere optischen Speichermedien wie CD-Rom, DVDs, optischen Bauteilen, Fenstermaterialien, usw.

Aus dem Stand der Technik sind verschiedene Methoden zur Detektion von lumineszierenden Partikeln in CD-Roms bekannt. Z.B. wird das fertige Kunststoffganulat geschmolzen, als CD verspritzt und auf Lumineszenz untersucht. In einer weiteren Methode wird das fertige Kunststoffsgranulat aufgelöst und die Lösung durch ein Sieb gefiltert. Letztlich werden die gefilterten Partikel mittels eines elektronischen Mikroskops bewertet.

Offensichtlich sind diese Methoden aufwändig und ermöglichen keine On-Line-Kontrolle im Produktionsprozess.

Es bestand daher Bedarf nach einem Mittel, das in einer Rohrleitung z.B. aus einer Produktionsanlage strömende Flüssigkeit in Echtzeit alle in einem vorgegebenen Messvolumen auftretenden lumineszierenden Partikel zuverlässig und genau detektieren kann. Dabei soll die Vorrichtung einfach und robust aufgebaut sein und insbesondere Temperaturen bis zu 400 °C bei einem Druck von 40 bar standhalten können.

WO 2006/136147 A2 beschreibt eine Vorrichtung zur Detektion von Streulichtpartikeln mit einer ticfenbegrenzten Lichtscheibe, bei der die in einem optischen begrenzten Messvolumen an der Vorrichtung vorbeilaufenden Partikel mittels einer Kamera erfasst werden. In dieser Vorrichtung wird in dem Messvolumen eine gleichmäßige Ausleuchtung ohne Lichtkonvergenz oder -divergenz erreicht, wobei das Messvolumen in der Tiefe mittels einer tiefenbegrenzten Lichtscheibe eng begrenzt ist, so dass nur die in diesem Volumen fließenden Partikel zu sehen sind. Eine Videokamera befindet sich orthogonal zur Lichtscheibe, über deren Auflösung nur die zwei Dimensionen der orthogonal zur Videokamera ausgerichteten Fläche des Messvolumens beschrieben werden. Durch eine schnelle, hochauflösende Bilderfassung und -speicherung mit Hilfe einer Auswertungssoftware ist sowohl eine Partikelzählung als auch eine Partikelidentifizierung möglich. Dabei muss zwischen zwei Bildern gewährleistet sein, dass das Messvolumen zu 100 % ausgetauscht wird. Eine Aufnahme der Partikel über eine längere Detektionszeit ist in WO 2006/136147 A2 vermieden, weil die Partiketzählung und -identifizierung nicht mehr zuverlässig wäre.

Da aber das von lumineszierenden Partikeln emittierte Licht üblicherweise eine geringe Intensität aufweist, arbeitet der Detektor oft an seiner Detektionsgrenze, so dass die sich bewegenden Partikel über eine längere Detektionszeit aufgenommen werden müssen.

Ausgehend von WO 2006/136147 A2 als nächstliegendem Stand der Technik bestand daher die Aufgabe darin, ein Mittel zu Detektion von lumineszierenden Partikeln in einer Rohrleitung bereit zu stellen, das das Unterscheiden zwischen dem von den lumineszierenden Partikeln emittierte Licht und dem Rauschen des Detektors ermöglicht.

Die Aufgabe wird durch eine Sonde zur Detektion von lumineszierenden und / oder lichtstreuenden Partikeln in strömenden Flüssigkeiten gelöst, die eine Messzelle umfassend folgende Elemente aufweist:
- einen Rohrleitungskanal, durch den die zu vermessende Flüssigkeit strömt,
- mindestens ein transparentes Fenster in einer Wand der Rohrleitung,
- mindestens eine Lichtquelle zur Erzeugung eines dimensionierten Anregungslichtstrahls, der durch das Fenster die lumineszierenden und / oder lichtstreuenden Partikel in dem Rohrleitungskanal in einem optisch begrenzten Messvolumen anregt,
- mindestens einen Detektor, der durch das Fenster oder durch ein weiteres Fenster elektromagnetische Strahlung von den lumineszierenden und / oder lichtstreuenden Partikeln aufnimmt,
wobei die Messzelle so aufgebaut ist, dass der dimensionierte Anregungslichtstrahl und das emittierte Licht senkrecht zueinander orientiert sind, und jedes Partikel sich innerhalb des Messvolumens parallel zum Flüssigkeitsstrom, geradlinig mit einem festen Winkel zum Anregungslicht bewegt.

Bei der vorliegenden Erfindung können die Partikel über eine längere Detektionszeit aufgenommen werden, während der sie sich in der strömenden Flüssigkeit weiterbewegen. Jedes Partikel weist eine gerichtete Strömung auf und kann über eine längere Detektionszeit als ein Lichtpunkt oder als eine gerichtete Lichtspur aufgenommen werden, was eine zuverlässige Bildanalyse ermöglicht.

Ein erster Gegenstand der vorliegenden Erfindung ist daher eine Sonde zur Detektion von lumineszierenden und / oder lichtstreuenden Partikeln in strömenden Flüssigkeiten, die eine Messzelle umfassend folgende Element aufweist:
- einen Rohrleitungskanal, durch den die zu vermessende Flüssigkeit strömt,
- mindestens ein transparentes Fenster in einer Wand der Rohrleitung,
- mindestens eine Lichtquelle zur Erzeugung eines dimensionierten Anregungslichtstrahls, der durch das Fenster die lumineszierenden und / oder lichtstreuenden Partikel in dem Rohrleitungskanal in einem optisch begrenzten Lichtvolumen anregt,
- mindestens einen Detektor, der durch das Fenster oder durch ein weiteres Fenster elektromagnetische Strahlung von den lumineszierenden und / oder lichtstreuenden Partikeln aufnimmt,
wobei die Messzelle so aufgebaut ist, dass der dimensionierte Anregungslichtstrahl und das emittierte Licht senkrecht zueinander orientiert sind und jedes Partikel sich innerhalb des Messvolumens parallel zum Flüssigkeitsstrom, geradlinig mit einem festen Winkel zum Anregungslicht bewegt.

Vorzugsweise liegt der feste Winkel der Partikel zum Anregungslicht im Bereich von 45 bis 135 Grad.

Um eine eindeutige Identifikation eines lumineszierenden Partikels anhand der Intensität seines Emissionslichts zu ermöglichen, wird üblicherweise diese Intensität über eine bestimmte Zeit - auch Integrationszeit genannt - aufsummiert. Die Integrationszeit definiert sich als die Zeit, die ein Partikel benötigt, um das Probevolumen bei einer festen Strömungsgeschwindigkeit zu durchströmen. Bei der vorliegenden Erfindung weist der Detektor entsprechend eine Schnittstelle zu einem Element zur Steuerung der Integrationszeit auf, so dass der Detektor das von den lumineszierenden Partikeln emittierte Licht über die Zeit aufnimmt, die ein Partikel benötigt, um das Lichtvolumen bei einer definierten Strömungsgeschwindigkeit zu durchströmen. Das Element zur Steuerung der Integrationszeit ist üblicherweise Teil eines Computers.

Typischerweise werden Rohrleitungen eines Durchmessers von 0,5 bis 50 mm, bevorzugt 4 bis 30 mm mit der erfindungsgemäßen Vorrichtung kontrolliert. Es ist dabei zu beachten, dass die Detektionsauflösung bei steigendem Rohrleitungsdurchmesser abnimmt. Entsprechend müssen die Lichtquellen und Detektoren an den Rohrleitungsdurchmcsser angepasst werden oder der Auflösungsverlust muss mit geeigneten Mittel wie z.B. hochauflösenden lichtempfindlichen Kameras, leistungsstarken Lichtquellen z.B. Laserlichtquellen oder Xenonlampen kompensiert werden.

Das Material der Rohrleitung ist beliebig, üblicherweise werden Rohrleitungen aus Metall verwendet.

Typischerweise werden als Lichtquelle zur Anregung von lumineszierenden Partikeln XenonLampen in Kombination mit Anregungsftltern, Laser mit geeigneter Emissionswelle oder Hochteistungs-LEDs verwendet.

Typischerweise werden die lumineszierenden Partikel mittels des Lichtstrahls bei einer Wellenlänge von 400 bis 500 nm angeregt.

Der von der Lichtquelle produzierte Anregungslichtstrahl wird üblicherweise durch ein Fenster eingebracht in der Rohrleitungswand über den kompletten Rohrleitungsdurchmesser des Rohrleitungskanals eingestrahlt. Die Dimensionen des Anregungslichtstrahls definieren das optisch begrenzte Messvolumen. Der besondere Vorteil dabei ist, dass durch die Bildaufnahme eines kleinen Abschnitts (Messvolumen) einer Rohrleitung über die Zeit der komplette Inhalt einer Rohrleitung erfasst werden kann. Bei Bedarf wird die Geometrie des Anregungslichtstrahls mit Hilfe von Zylinderlinsen oder LichEleiterquerschnittswandlern gestaltet.

Üblicherweise wird die senkrechte Orientierung des dimensionierten Anregungslichtstrahls zu dem von den lumineszierenden Partikeln emittierten Licht durch eine senkrechte Orientierung der Lichtquelle und des Detektors zueinander gewährleistet. Alternativ kann mittels Prismen und Spiegeln die nötige Orientierung der jeweiligen Lichtstrahlen zueinander erreicht werden.

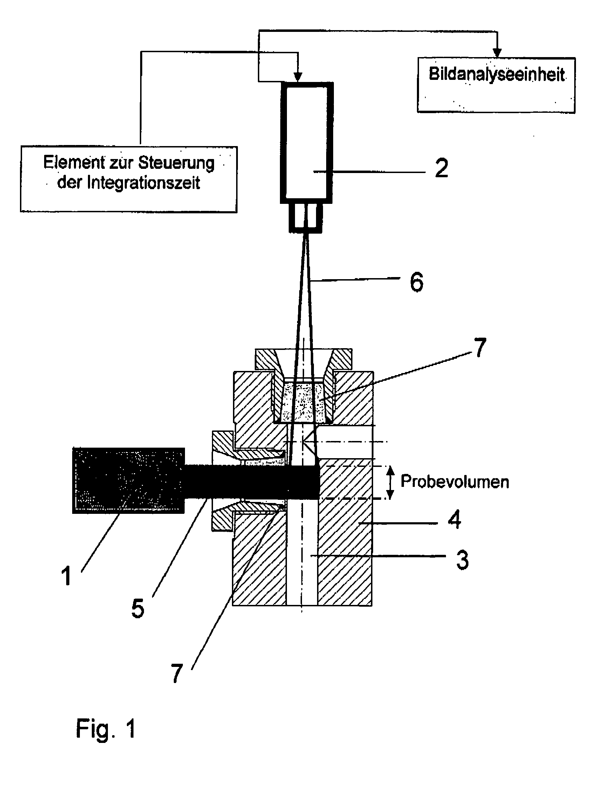

In einer ersten Ausführungsform der erfindungsgemäßen Sonde befinden sich in der Rohrleitungswand ein transparentes Fenster zur Beleuchtung des Rohrleitungskanals (Beleuchtungsfenster) mit dem Anregungslicht und ein weiteres transparentes Fenster zur Aufnahme des Einissionslichts mittels des Detektors (Detektionsfenster). In dieser besonderen Ausführungsform (siehe Fig. 1) ist die Rohrleitung in einem Winkel von 90° geknickt. Das βeleuchiungsfenster befindet sich an einer Seite der Rohrleitung vor dem Knick und das Detektionsfenster befindet sich an der Seite der Rohrleitung unmittelbar nach dem Knick, so dass das Detektionsfenster über dem unteren Teil des Rohrteitungsleitungskanals offen ist und der Detektor den zu sich fließenden Flüssigkeitsstrom aufnimmt. Diese Ausführungsform hat den besonderen Vorteil, dass der Strom in einem festen Winkel von 0 Grad zur Strömungsrichtung beobachtet wird, und dass jedes Partikel entsprechend als Punkt detektiert wird, vorausgesetzt es bewegt sich während der kompletten lntegrationszeit geradlinig senkrecht zum Anregungslicht.

Bei dieser Ausführungsform der Erfindung ist es vorteilhaft, wenn das Lichtvolumen maximal doppelt so hoch wie der Tiefenschärfenbereieli des Detektors ist, typischerweise wird der Anregungslichtstrahl auf einen Durchmesser von 100 µm bis 10 mm, bevorzugt 150 µm bis 3 mm fokussiert. Ist das Messvolumen größer als der Tiefenschärfenbereich, werden die Partikel nicht mehr exakt vermessen. Ist lediglich eine Detektion von Ereignissen gefordert, sollte das Messvolumen so groß wie nur möglich sein, um möglichst viel Licht zu sammeln.

Da der Winkel der Rohrleitung die Richtung des Flüssigkeitsstroms in der Leitung auch vor dem Knick beeinflusst, ist es vorteilhaft, wenn der Aufbau der Messzelle das laminare Strömen der Partikel unbehindert innerhalb des Messvolumens, d.h. ohne tote Räume und bei konstanter Geschwindigkeit, geradlinig unterstützt. Hierfür können verschiedene Mittel einzeln oder miteinander kombiniert verwendet werden.

Es ist z.B. bevorzugt, dass das Fensterglas in die Rohrleitungswand bündig mit dem Rohrleitungskanal befestigt ist. Die Form des Fensters ist beliebig, üblicherweise rund mit einem Durchmesser von 2 bis 100 mm. Alternativ kann eine Sonde aus Saphir oder Quarzglas angefertigt werden, die an die Rohrleitung befestigt wird.

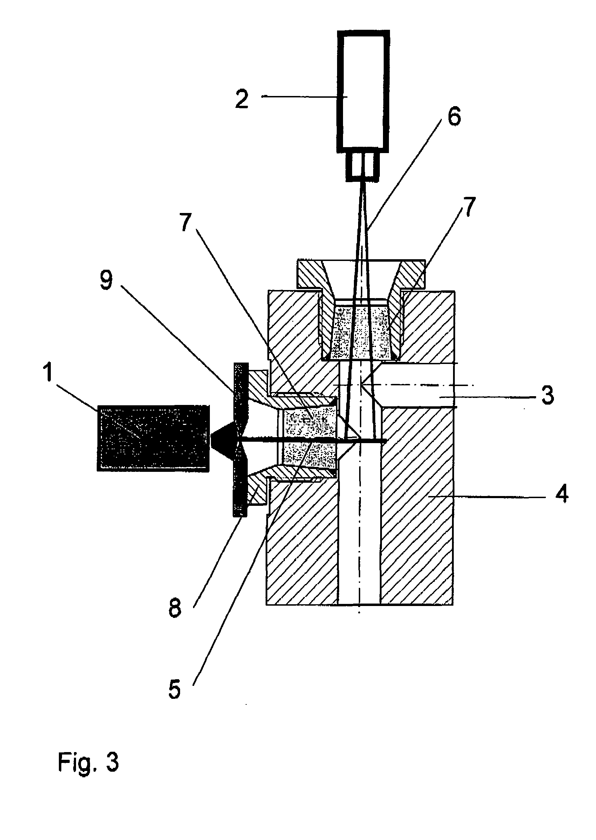

Für die Anwendbarkeit in einer Kunststoffsproduktionsanlage müssen die Fenster dem Strömen einer Schmelze bei einer Temperatur von bis zu 400 °C und einem Druck von I bis 250 bar standhalten. Typischerweise besteht das Fenster aus Saphir bzw. Quarzglas, bevorzugt Saphir wegen seiner besonderen Festigkeit, weist eine Dicke von 10 mm auf und ist - wie z.B. in DE 102 01 541 A1 beschrieben - konisch geformt. Durch Druck mittels einer Glas-Metall-Dichtung kann das Fensterelement in die Rohrleitungswand bündig mit dem Rohrleitungskanal befestigt werden (Fig. 3).

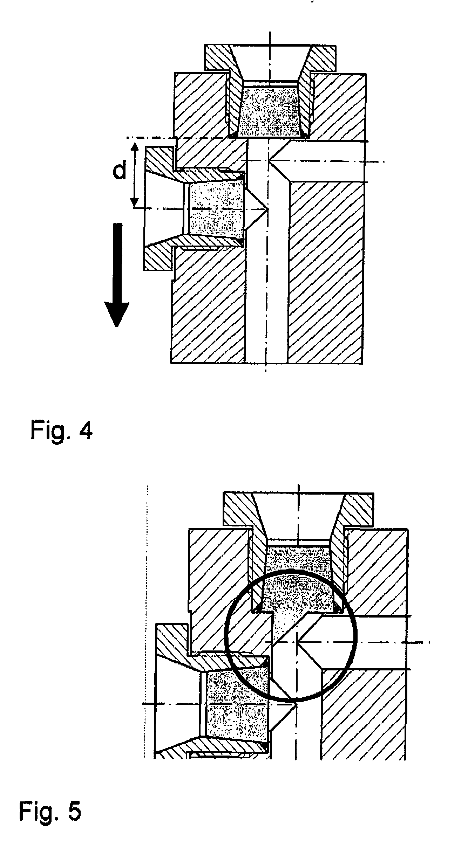

Es ist außerdem bevorzugt, dass die Distanz d ab dem Zentrum des Beleuchtungsfensters und die Oberfläche des Detektionsfenster für ein optimales Strömen der Partikel an die Größe der Rohrleitung angepasst wird (Fig. 4). Je nach Anwendungsbereich ist es außerdem vorteilhaft, die Distanz d an die die Strömungsgeschwindigkeit und die Viskosität der untersuchten Flüssigkeit anzupassen, um das laminare Strömen innerhalb des Messbereiches zu optimieren.

Es ist auch möglich, das Detektionsfenster so zu gestalten, dass tote Räume in dem 90°-Winkel der Rohrleitung minimal sind. Hierfür kann der Aufbau des Detektionstensters angepasst werden wie z.B. in Fig. 5 dargestellt.

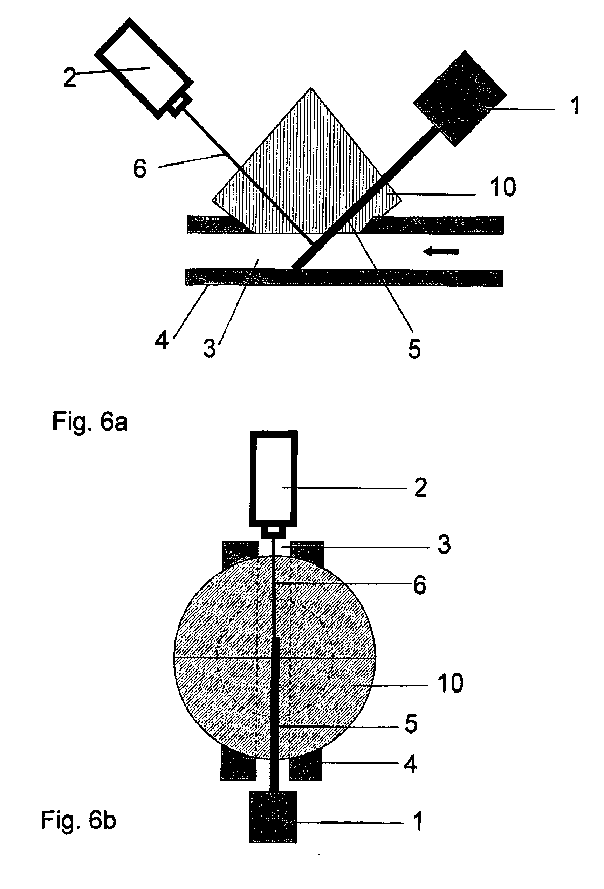

In einer zweiten Ausführungsform der erfindungsgemäßen Sonde wird die nötige Orientierung der jeweiligen Lichtstrahlen zueinander mittels eines Prismas erreicht. Üblicherweise weist dann die Messzelle ein einziges Fenster auf, das am Rand der Rohrleitung in der Rohrleitungswand eingesetzt ist und als Fensterglas das Prisma aufweist (Fig. 5 und 6). Alternativ kann eine Sonde aus Saphir oder Quarzglas mit der passenden Prisma-Geometrie angefertigt werden, die an die Rohrleitung befestigt wird.

Diese besondere Ausführungsform hat den Vorteil, dass der Flüssigkeitsstrom unbehindert an dem Fenster vorbei fließen kann. Die Positionierung der Lichtquelle, des Detektors und die Geometrie und optischen Eigenschaften des Prismas sichern die passende senkrechte Orientierung des Anregungslichts zu dem Emissionslicht. Beobachtet wird in einem festen Winkel von bevorzugt 45° bzw. 135° zur Strömungsrichtung. Bei dieser Ausführung wird das Partikel als gerichteter Strich aufgenommen.

Bei der Prismenausführung ist die Dicke des Anregungslichtstrahls vorzugsweise dünner als der Durchmesser der Rohrleitung. Vorteilhaft ist eine Dicke von maximal 5 mm, die aber von dem Durchmesser des Strömungskanal abhängt. Beispielsweise wird für einen Strömungskanaldurchmesser von 5 mm eine Lichtstrahldicke von maximal 1 mm bevorzugt. Ist das Messvolumen größer als der Tiefenschärfenbereich, werden die Partikel nicht mehr exakt vermessen. Ist lediglich eine Detektion von Ereignissen gefordert, sollte das Messvolumen so groß wie nur möglich sein, um möglichst viel Licht zu sammeln.

Für die beschriebenen Ausführungsformen 1 und 2 kann es vorteilhaft sein, die Messzelle direkt mittels Heizungselemente zu temperieren, so dass die Temperatur der vorbei strömenden Flüssigkeit konstant gehalten werden kann. Typische Heizungselemente sind Ölbegleitheizung über Heizkanäle oder elektrische Beheizung.

Bei der vorliegenden Erfindung kann der Detektor die Intensität des von den lumineszierenden Partikeln emittierten Lichts üblicherweise bei einer Wellenlänge von 500 bis 700 nm registrieren. Wird die Intensität des von den lichtstreuenden Partikeln emittierten Lichts vom Detektor registriert, findet dies üblicherweise bei der Anregungswettenlänge statt. Gegebenenfalls werden Emissionsfilter eingesetzt, um diesen Wellenlängenbereich selektiv zu erfassen.

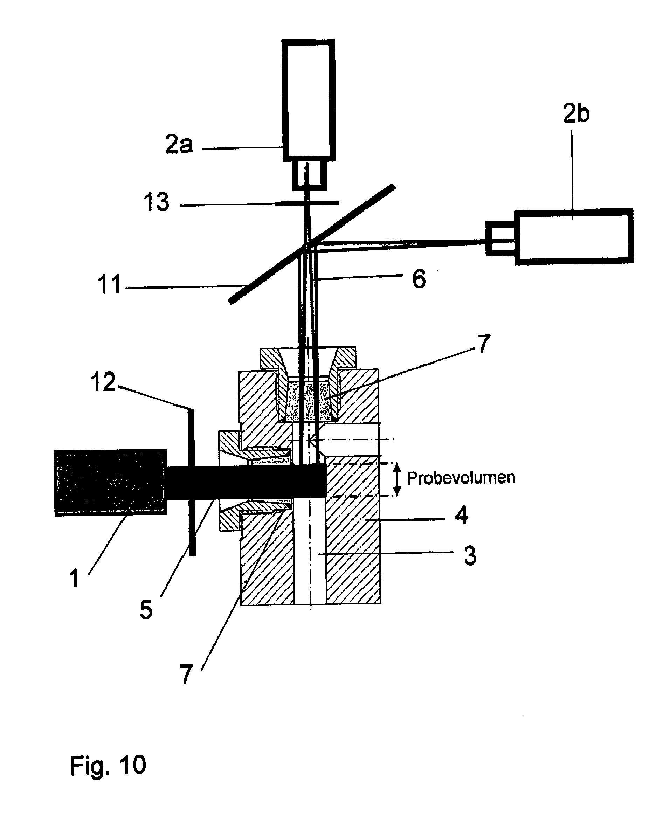

Es ist auch möglich, mehrere Detektoren einzusetzen, wobei Detektoren zur Detektion von lumineszierenden Partikeln und Detektoren zur Detektion von lichtstreuenden Partikeln kombiniert werden können (z.B. wie in Fig. 10 dargestellt).

Mögliche Detektoren sind beispielsweise CCD-Kameras, CMOS-Kameras, Verstärkerkameras, Photomultiplier, Photozellen. Geeignete Kameras sind welche, die in dem Detektionswellenbereich (500-700 nm) ausreichend lichtempfindlich sind. Beispielsweise wird die Kamera Stingray von der Firma AVT verwendet. Der Vorteil einer Kamera besteht darin, dass nicht nur die Lumineszenzintensität der Partikel sondern auch ihre Fläche erfasst werden kann.

Erfindungsgemäß strahlt die Lichtquelle kontinuierlich oder über die Integrationszeit das Probevolumen des Strömungskanals an und regt die vorbei fließenden Partikel an.

Üblicherweise wird die Integrationszeit an die Größe des Probenvolumens und an die Strömungsgeschwindigkeit angepasst.

Der Detektor nimmt über die Integrationszeit das Emissionslicht aus dem Kanalinneren auf und leitet diese Information an eine Bildanalyseeinheit, welche üblicherweise Teil eines Computers ist, weiter.

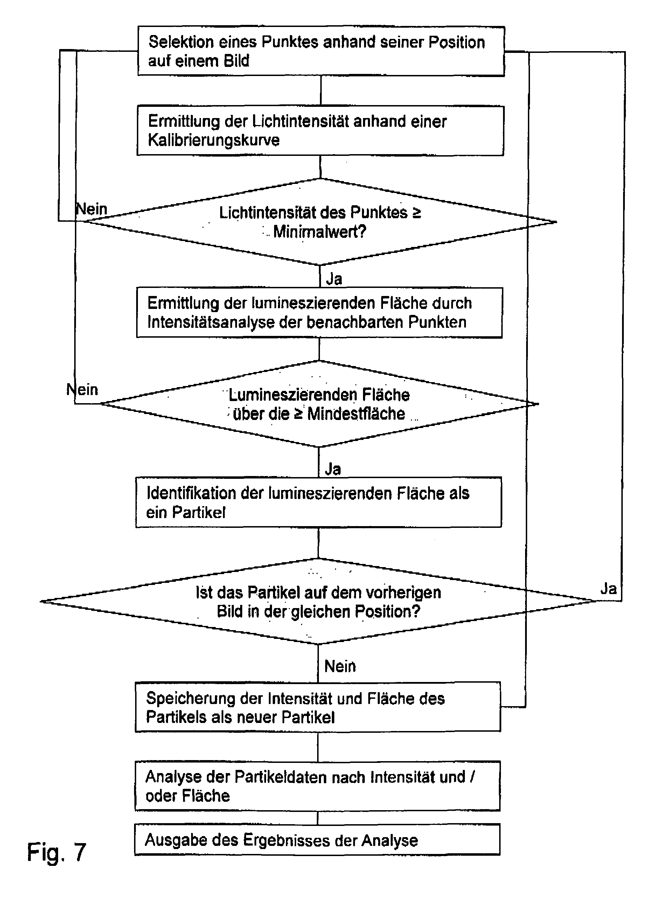

Die Analyse des Bildmaterials erfolgt typischerweise nach dem Diagramm der Fig. 7, die Daten werden bewertet und ausgegeben.

Ein weiterer Gegenstand der vorliegenden Erfindung ist daher ein Verfahren zur Detektion von iumincszierenden und / oder liehtstreuenden Partikeln in strömenden Flüssigkeiten mit folgenden Schritten:
- Eingabe der Höhe des Probevolumens und Eingabe der Strömungsgeschwindigkeit sowie Berechnung der Integrationszeit in ein Element zur Steuerung der Integrationszeit,
- Lichtanregung durch eine Lichtquelle,
- Detektion von Emissionsstrahlung über die lntegrationszeitmittels eines Detektors,
- Analyse der Detektionsdaten mittels einer Bildanalyseeinheil
- Ausgabe der Anzahl von Partikeln und/ oder Größenverteilung von Partikein und/ oder Intensitätsverteilung von Partikeln pro Volumen und / oder pro Gewicht und /oder Ausgabe eines Sammelbildes von lumineszierenden Partikeln über eine bestimmte Zeit.

Ein weiterer Gegenstand der vorliegenden Erfindung ist die Verwendung der erfindungsgemäßen Sonde und / oder des erfindungsgemäßen Verfahrens zur Online-Überwachung einer Produktionsanlage, insbesondere Kunststoffsproduktionsanlage, Kläranlage.

Die Figuren 1, 3 bis 6 zeigen mögliche Ausführungen der erfindungsgemäßen Vorrichtung, ohne sie darauf zu begrenzen.

Figur 2 und 7 zeigen den Ablauf des erfinderischen Verfahrens und den Ablauf der Bildanalyse in der Bildanalyseeinheit, ohne es darauf zu begrenzen.

### Figuren:

- Fig. 1:: erfindungsgemäße Sonde anhand der Ausführungsfortu 1

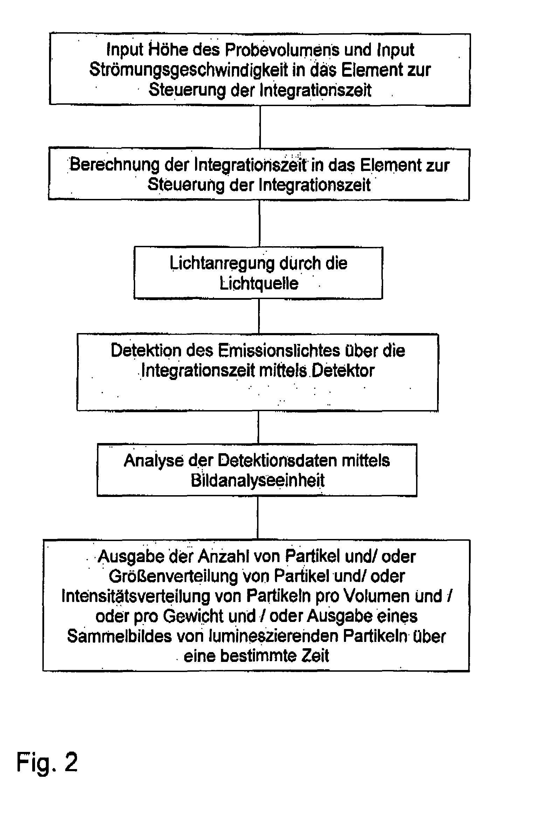

- Fig.2:: Verfahrensdiagramm
- Fig. 3:: Ausführungsform 1
- Fig. 4:: Optimierung des Distanz d bei der Ausführungsform 1
- Fig. 5:: Fenstervariante der Ausführungsform 1
- Fig. 6a:: Seitenansicht der Ausführungsform 2 mit dem Prisma
- Fig. 6b:: Sicht von oben der Ausführungsform 2 mit dem Prisma
- Fig. 7:: Diagramm der Bildanalyse in der Bildanalyseeinheit

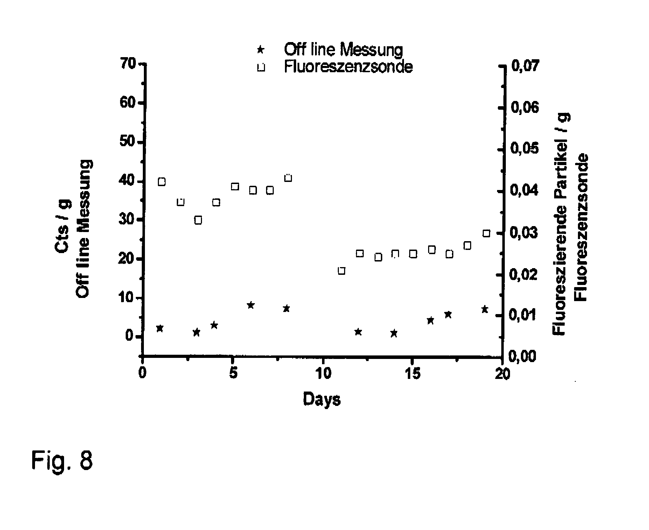

- Fig. 8:: Ausgabe der Anzahl von fluoreszierenden Partikeln pro Gramm Schmelze über die Zeit

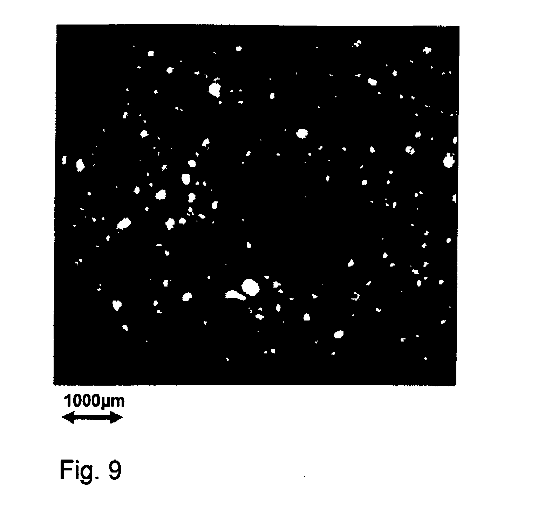

- Fig. 9:: Sammelbild der fluoreszierenden Partikeln über 6 Stunden.
- Fig. 10:: Sonde zur gleichzeitigen Detektion von lumineszierenden Partikeln und lichtstreuenden Partikeln

### Bezugzeichen:

- 1: Lichtquelle
- 2: Detektor
- 2a: Detektor zur Detektion von lumineszierenden Partikeln
- 2b: Detektor zur Detektion von lichtstreuenden Partikeln
- 3: Rohrleitungskanal
- 4: Rohrleitungswand
- 5: Anregungslichtstrahl
- 6: Emissionslicht
- 7: Fensterglas
- 8: Glas-Metall-Dichtung
- 9: Apertur
- 10: Prisma
- 11: Dichriotischer Spiegel 530nm
- 12: Anregungsfilter 400-500nm
- 13: Fluoreszenzfilter 550-650nm

### Beispiel:

Eine Rohrleitung mit einem Rohrleitungskanal von 8 mm Durchmesser wurde in einem 90°-Winkel geknickt.

In der Rohrleitungswand wurden an einer Seite der Rohrleitung vor dem Knick ein Beleuchtungsfenster und an der Seite der Rohrleitung unmittelbar nach dem Knick ein Detektionsfenster gefräst, so dass das Detektionsfenster über dem unteren Teil des Rohrleitungsleitungskanals offen war und der Detektor den zu sich fließenden Flüssigkeitsstrom aufnehmen konnte.

Die Distanz d ab dem Zentrum des Beleuchtungsfensters und die Oberfläche des Detektionsfensters betrug 14 mm.

Die Fenster waren beide rund mit einem Durchmesser von 9 mm. In jedem Fenster wurde ein 10 mm dickes konisch geformtes Fensterglas aus Saphir bündig mit dem Rohrleitungskanal durch Druck mittels einer Glas-Metall-Dichtung befestigt (Fig. 3).

Die Sonde wurde in die Rohrleitung einer Polycarbonatanlage eingebaut, in der eine Polycarbonatschmelze bei einer Temperatur von 300 °C mit einer Strömungsgeschwindigkeit von 6 m / min floss.

Vor dem Beleuchtungsfenster wurden eine kommerziell verfügbare Xenon Lampe (Drelloscop 255, der Firma Drello) in Kombination mit Anregungsfilter (HQ450/100 M-2P LOT Oriel) sowie eine Apertur befestigt Die Anregungswcllcnlänge des Lichtstrahls wurde mit Hilfe des Anregungsfilters auf 400-500 nm eingestellt. Der Lichtstrahl wurde auf einem Durchschnittsdurchmesser von 2 mm mittels der Apertur fokussiert.

Vor dem Detektionsfenster wurde eine Kamera (Stingray von der Firma AVT) in Kombination mit einem Emissionsfilter (HQ600/100M-2P der Firma LOT Oriel) und eines Strahlteilers (530DCXRU der Firma LOT Oriel) zur Selektion der Aufnahme in einem Wellenlängenbereich von 550 bis 650 nm befestigt. Die Kamera wurde senkrecht zum Anregungslicht befestigt, so dass sie den kompletten Durchmesser des Rohrleitungskanals aufnehmen konnte.

Die Schnittstelle der Kamera wurde mit einem Element zur Steuerung der Integrationszeit und mit einer Bildanalyseeinheit, beide Elemente eines Computers, verbunden.

In dem Element zur Steuerung der Integrationszeit wurden die Höhe des Probevolumens (2 mm) und die Strömungsgeschwindigkeit eingegeben. Eine lntegrationszeit von 20 ms wurde berechnet.

Die Lichtquelle beleuchtete das Probenvolumen bei einer Wellenlänge von 400-500 nm kontinuierlich.

Die Kamera nahm Bilder des Probenvolumen in einem Detektionswellenlängenbereich von 550 bis 650 nm über die Integrationszeit gesteuert durch das Element zur Steuerung der Integratiotis₇eit auf.

Die aufgenommenen Daten wurden von der Kamera in die Bildanalyseeinheit übertragen und wurden von der Bildanalyseeinheit gemäß Fig. 7 bearbeitet.

Die Figuren 8 und 9 zeigen mögliche Ausgaben nach Bearbeitung der Daten.

## Patentansprüche

1. Sonde zur Detektion von lumineszierenden und / oder lichtstreuenden Partikeln in strömenden Flüssigkeiten, die eine Messzelle beinhaltend folgende Elemente aufweist:
- ein Rohrleitungskanal, durch das die zu vermessende Flüssigkeit strömt,
- mindestens ein transparentes Fenster in einer Wand der Rohrleitung,
- mindestens eine Lichtquelle zur Erzeugung eines dimensionierten Anregungslichtstrahls, der durch das Fenster die lumineszierenden und / oder lichtstreuenden Partikeln in dem Rohrleitungskanal in einem optisch begrenzten Lichtvolumen anregt,
- mindestens einen Detektor, der durch das Fenster oder durch ein weiteres Fenster elektromagnetische Strahlung von den lumineszierenden und / oder lichtstreuenden Partikeln aufnimmt,
wobei die Messzelle so aufgebaut ist, dass der dimensionierte Anregungslichtstrahl und das von den lumineszierenden und / oder lichtstreuenden Partikeln emittierte Licht senkrecht zueinander orientiert sind und jedes Partikel sich innerhalb des Messvolumens parallel zum Flüssigkeitsstrom, geradlinig mit einem festen Winkel zum Anregungslicht bewegt.

2. Sonde nach Anspruch 1 **dadurch gekennzeichnet, dass** der feste Winkel der Partikel zum Anregungslicht im Bereich von 45 bis 135 Grad liegt.

3. Sonde nach einem der Ansprüche 1 oder 2, **dadurch gekennzeichnet, dass** der Detektor eine Schnittstelle zu einem Element zur Steuerung der lntegrationszeit aufweist, so dass der Detektor das von den lumineszierenden Partikeln emittierte Licht über die Zeit aufnimmt, die ein Partikel benötigt, um das Lichtvolumen bei einer definierten Strömungsgeschwindigkeit zu durchströmen.

4. Sonde nach einem der Ansprüche 1 bis 3 **dadurch gekennzeichnet, dass** der Anregungslichtstrahl über den kompletten Rohrleitungsdurchmesser des Rohrleitungskanals einstrahlt.

5. Sonde nach einem der Ansprüche 1 bis 4 **dadurch gekennzeichnet, dass** die Rohrleitung in einem 90°-Winkel geknickt ist und die Rohrleitung an einer Seite vor dem Knick ein transparentes Beleuchtungsfenster zur Beleuchtung des Rohrleitungskanals und an der Seite der Rohrleitung unmittelbar nach dem Knick ein transparentes Detektionsfenster zur Aufnahme des Emissionslichts mittels des Detektors aufweist, so dass das Detektionsfenster über dem unteren Teil des Robrleitungsleitungskanals offen ist und der Detektor den zu sich fließenden Flüssigkeitsstrom aufnimmt.

6. Sonde nach Anspruch 5 **dadurch gekennzeichnet, dass** die Distanz d ab dem Zentrum des Beteuchtungsfensters und die Oberfläche des Detektionsfensters für ein optimales Strömen der Partikel an die Größe der Rohrleitung angepasst ist.

7. Sonde nach einem der Ansprüche 5 oder 6 **dadurch gekennzeichnet, dass** das Lichtvolumen maximal genau so hoch wie der doppelte Tiefenschärfebereich des Objektivs ist.

8. Sonde nach einem der Ansprüche 1 bis 4 **dadurch gekennzeichnet, dass** die Messzelle ein einziges Fenster aufweist, das am Rand der Rohrleitung in der Rohrleitungswand eingebracht ist und als Fensterglas ein Prisma aufweist, das die senkrechte Orientierung des Anregungslichts zu dem Emissionslicht gewährleistet.

9. Sonde nach Anspruch 8 **dadurch gekennzeichnet, dass** die Dicke des Anregungslichtstrahls maximal 5mm beträgt.

10. Sonde nach einem der Ansprüche 1 bis 9, die ein Detektor für lumineszierende Partikel und ein Detektor für Streulichtpartikel aufweist.

11. Verfahren zur Detektion von lumineszierenden und / oder lichtstreuenden Partikeln in strömenden Flüssigkeiten mit folgenden Schritten:
a. Eingabe der Höhe des Probevolumens und Eingabe der Strömungsgeschwindigkeit sowie Berechnung der Integrationszeit in ein Element zur Steuerung der integrationszeit,
b. Lichtanregung durch eine Lichtquelle,
c. Detektion des Emissionslichtes über die Integrationszeit mittels eines Detektors,
d. Analyse der Detektionsdaten mittels einer Bildanalyseeinheit
e. Ausgabe der Anzahl von Partikeln und/ oder Größenverteilung von Partikeln und/ oder Intensitätsverteilung von Partikeln pro Volumen und / oder pro Gewicht und / oder Ausgabe eines Sammelbildes von lumineszierenden oder lichtstreuenden Partikeln über eine bestimmte Zeit.

12. Verwendung der Sonde nach einem der Ansprüche 1 bis 10 oder des Verfahrens nach Anspruch 11 zur Online-Überwachung einer Produktionsanlage, insbesondere einer Kunststoffsproduktionsanlage oder einer Kläranlage.
